# EUROPEAN PATENT APPLICATION

(11) **EP 4 697 351 A1**
(43) Date of publication of application: **18.02.2026**
(21) Application number: 24197039.1
(22) Date of filing: 28.08.2024
(51) Int. Cl.: G16H 40/63, G16H 15/00, G16H 50/20, G16H 50/30, A61B 5/00

(54) **SUBJECT MONITORING**

(30) Priority: 14.08.2024 US 202463682942 P
(71) Applicant: Koninklijke Philips N.V., 5656 AG Eindhoven (NL)
(72) Inventor: HUIJBREGTS, Laurentia Johanna, 5656 AG Eindhoven (NL); ZHU, Di, 5656 AG Eindhoven (NL); BEAULIEU, Tammy, 5656 AG Eindhoven (NL)
(74) Representative: Philips Intellectual Property & Standards

(57) **Abstract**

Proposed concepts thus aim to provide schemes, solutions, concepts, designs, methods and systems pertaining to a method for controlling a display. In particular embodiments aim to provide a method for controlling a display comprising modifying a display control signal if a predetermined condition is met.

## Description

### FIELD OF THE INVENTION

This invention relates to the field of subject monitoring, and more specifically, to the field of the controlling subject monitoring displays.

### BACKGROUND OF THE INVENTION

Subject monitoring displays are used in clinical practice to provide visual representations of subject vital signs through color, shape, and animation, to deliver an overview of a subject's health at a glance. The use of such displays may have many clinical advantages and result in an improved level of care for subjects by aiding clinicians to make better health decisions.

Many different clinical values describing the current and/or past health status of a subject may be available. These typically include a mixture of real-time measurements of subject health parameters and historic data describing the subject's condition in the past, alongside other subject parameters such as the subject's age, demographic characteristics, past diagnoses, and details of administered treatments.

In order to not overwhelm the clinical practitioner with large amounts of data at once, only a subset of the total medical information available for a subject may be presented on a subject monitoring display at a given time. Thus, the clinician may be typically required to manually scan through different displays or select which information they wish to see in order to perform a full and accurate assessment of the subject's health.

### SUMMARY OF THE INVENTION

The invention is defined by the claims.

According to examples in accordance with an aspect of the invention, there is provided a method for controlling a display.

The method comprises: generating a first display control signal for controlling a display, the first display control signal describing a first avatar of the subject, wherein the first avatar visually indicates a first set of clinical values of the subject; determining whether a predetermined condition is met based on a second set of clinical values of the subject, wherein the second set of clinical values describe a physiological subsystem of the subject; and if the predetermined condition is met, modifying the first display control signal to generate a second display control signal, the second display control signal describing: a modification to the first avatar; and a second avatar of the subject wherein the second avatar visually indicates a third set of clinical values describing the physiological subsystem of the subject.

Proposed concepts thus aim to provide schemes, solutions, concepts, designs, methods and systems pertaining to a method for controlling a display. In particular, embodiments aim to provide a method for controlling a display comprising modifying a display control signal if a predetermined condition is met.

The proposed method therefore permits the automatic control of a subject monitoring display to show two different displays. For general monitoring of a subject, it may be preferable to show an overview of the subject's health including vital signs measurement and particularly important holistic clinical values. However, in certain clinical situations more specific clinical values providing a more detailed description of a certain physiological subsystem of the subject may be required in order for a clinician to make treatment decisions. For example, if a particular physiological subsystem of the subject is experiencing a rapid deterioration in condition, the clinician may require detailed information of this subsystem to assess what action to take to improve the subject's condition.

In the proposed method, the first display control signal controls the subject monitoring display to show a first avatar of the subject which visually indicates a first set of clinical values of the subject. The first avatar may be used for general vital signs monitoring of the subject. Based on analysis of a set of clinical values that relate to a specific physiological subsystem of the subject (for example a set of clinical values describing the cardiac or pulmonary system of the subject), the first display control signal is modified to generate a second display control signal. The second display control signal comprises information about a second set of clinical values that describe the physiological subsystem in more detail.

Thus, the proposed methods and systems permit the automatic switching between different views on a subject monitoring display. This results in different clinical values being presented to a health care professional depending on the current condition of the subject. Embodiments thus advantageously result in the priority of the display of certain data to a clinician to allow for more efficient and accurate subject monitoring.

In some embodiments, an avatar may comprise a graphical representation of at least one body part of the subject. This may provide context to each displayed clinical value and therefore improve the ease with which a clinical professional can see and assess these values.

In some embodiments, the first avatar may comprise a graphical representation of the subject's whole body and the second avatar may comprise a graphical representation of the physiological subsystem of the subject. This permits easy visual distinction between the first set of clinical values and the third set of clinical values.

In some embodiments, a modification of the first avatar may comprise at least one of: a modification to be made to a graphical property of the first avatar; and a removal of the first avatar. Either of these actions would result in greater focus on the second avatar in response to the first condition being met indicating the particular importance of the third set of clinical values.

In some embodiments, the graphical property of the first avatar may comprise at least one of: a dimension of the first avatar; a scale of the first avatar; a position of a first avatar; an orientation of the first avatar; a color of the first avatar; a color of a perimeter region of the first avatar; and a transparency of the first avatar. Modifying any of these properties of the first avatar can be used to emphasize the relative importance of the first and third set of clinical values.

In some embodiments, determining whether a predetermined condition is met may comprise: processing the second set of clinical values with a predictive model to predict a future condition of the physiological subsystem; and determining whether the predetermined condition is met based on the predicted future condition. Thus, the method may modify the display control signal based on a prediction that the subject's condition will change/deteriorate at some point in the future. This may allow for improved monitoring of the subject's health condition.

In some embodiments, determining whether a predetermined condition is met may comprise: comparing a clinical value of the second set of clinical values to a predetermined threshold value; and determining whether the predetermined condition is met based on this comparison. Certain clinical values falling below or rising above certain threshold values may indicate dangerous or critical subject states. Modifying the display based on comparison of clinical values to predetermined threshold values may therefore result in accurate and timely alerts to a clinical professional of a change in a subject's condition.

In some embodiments, determining whether a predetermined condition is met may comprise: analyzing the second set of clinical values to determine the rate of change of a value of a parameter of the physiological subsystem; and determining whether the predetermined condition is met based on the determined rate of change of the value of the parameter of the physiological subsystem. Rapid changes in clinical values may indicate certain health conditions. This embodiment therefore permits a subject monitor to indicate when the condition of the subject is no longer stable.

In some embodiments, the first set of clinical values may comprise a plurality of vital sign measurements of the subject and optionally the plurality of vital sign measurements may comprise at least one of: a blood oxygen saturation value (SpO2); a heart rate value; a respiration rate value; an ST elevation value; a blood pressure value; a central venous pressure value; an end-tidal carbon dioxide value; a body temperature value; a cardiac output value; a brain activity value; a relaxation status value; an fraction of inspired oxygen (FiO2) value; an airway pressure value; and a tidal volume. The display of these values permits efficient and accurate monitoring of the subject's health status.

In some embodiments, the second set of clinical values may comprise a subset of the third set of clinical values. Thus, the clinical values represented by the second avatar include those used in the determination of whether the predetermined condition is met and further values describing the same physiological subsystem. Thus, the second avatar provides information about the physiological subsystem that may be useful for subject assessment and treatment.

In some embodiments, the second set of clinical values may describe at least one time varying parameter of the physiological subsystem and determining whether a predetermined condition is met comprises determining whether the predetermined condition is met during a first time period. Thus, the proposed method may advantageously permit real-time assessment of the subject's state.

In some embodiments, if the predetermined condition is met in the first time period, the method may further comprise: determining, based on the second set of clinical values, whether the predetermined condition is met in a second time period that commences later than the first time period; and if the predetermined condition is not met in the second time period, modifying the second display signal to generate a third control display signal describing a modification to the second avatar, and the first avatar of the subject. Thus, there is also provided a method for switching back to the first avatar once the subject has stabilized to allow for more general monitoring of the subject.

According to another aspect, there is provided a computer program comprising computer program code means adapted, when said computer program is run on a computer, to implement the method of any of the above described embodiments.

According to yet another aspect there is provided a processing arrangement configured to: generate a display control signal for controlling a display, the display control signal describing a first avatar of the subject, wherein the first avatar visually indicates a first set of clinical values of the subject; determine whether a predetermined condition is met based on a second set of clinical values of the subject, wherein the second set of clinical values describe a physiological subsystem of the subject; and if the predetermined condition is met, modify the first display control signal to generate a second display control signal, the second display control signal describing a modification to the first avatar, and a second avatar of the subject, wherein the second avatar visually indicates a third set of clinical values describing the physiological subsystem of the subject.

There is further provided a system for displaying clinical values, the system comprising: a display; and the processing arrangement described above for controlling the display.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a simplified flow diagram of a computer-implemented method for controlling a display according to a proposed embodiment.
Fig. 2 is a simplified flow diagram of a computer-implemented method for controlling a display according to an alternative proposed embodiment.
Fig. 3A, 3B, and 3C are each schematic illustrations of a subject monitoring display controlled according to a proposed method of the present invention;
Fig. 4 is a simplified flow diagram of a computer-implemented method for controlling a display according to a third proposed embodiment;
Fig. 5 is a simplified block diagram of a system for displaying clinical values of a subject according to an aspect of the present invention; and
Fig. 6 illustrates an example of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems, and methods, are intended for purposes of illustration only and are not intended to limit the scope of the invention. These and other features, aspects, and advantages of the apparatus, systems, and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Implementations in accordance with the present disclosure relate to various techniques, methods, schemes and/or solutions pertaining to controlling a display. According to proposed concepts, a number of possible solutions may be implemented separately or jointly. That is, although these possible solutions may be described below separately, two or more of these possible solutions may be implemented in one combination or another.

Subject monitoring displays can be used to represent a selection of the subject's vital signs to a clinical professional. For general monitoring it may be beneficial to display only a small selection of crucial clinical values of the subject to allow for a quick assessment of the subject's condition at a glance. However, in certain situations, this selection may not be sufficient and the clinician may require more information to perform a more detailed clinical assessment of the subject. The display control method proposed by this invention results in the automatic determination of whether the information displayed on a subject monitoring display is sufficient and modifying a display control signal to modify the information shown on the subject monitoring display to show different/additional information if the current display is deemed insufficient. The proposed invention may therefore permit improved subject monitoring and care.

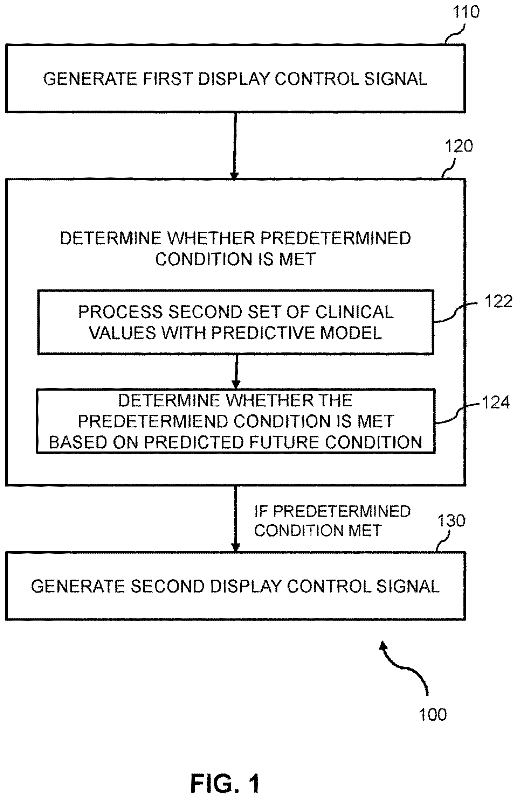

Referring now to Fig. 1 there is depicted a simplified flow diagram of a method 100 for controlling a display according to a proposed embodiment.

The method commences with a step 110 comprising generating a first display control signal for controlling a display, the first display control signal describing a first avatar of the subject, wherein the first avatar visually indicates a first set of clinical values of the subject. In this exemplary embodiment, the first avatar comprises a graphical representation of the subject's whole body and the first set of clinical values comprise a plurality of vital signs measurements of the subject. The plurality of vital sign measurements comprise at least one of: a blood oxygen saturation value (SpO2); a heart rate value; a respiration rate value; an ST elevation value; a blood pressure value; a central venous pressure value; an end-tidal carbon dioxide value; a body temperature value; a cardiac output value; a brain activity value; a relaxation status value; a fraction of inspired oxygen (FiO2) value; an airway pressure value; and a tidal volume. These are just several examples of values that may be represented by the first avatar, the first set of clinical values may describe other physiological parameters in other embodiments.

The method then proceeds to step 120 comprising determining whether a predetermined condition is met based on a second set of clinical values of the subject, wherein the second set of clinical values describe a physiological sub-system of the subject. In this example, the physiological sub-system of the subject is the subject's cardiac system, and the second set of clinical values is a set of cardiac parameters of the subject.

In this exemplary embodiment, step 120 comprises two sub-steps. Step 122 comprises processing the second set of clinical values with a predictive model to predict a future condition of the physiological subsystem. Step 124 comprises determining whether the predetermined condition is met based on the predicted future condition. In this way the predictive model uses clinical values of the subject to predict the likelihood that the condition of the subject's physiological subsystem may change at some point in the future.

If it is determined that the predetermined condition is met, the method proceeds to step 130 comprising modifying the first display control signal to generate a second display control signal, the second display control signal describing a modification to the first avatar, and a second avatar of the subject wherein the second avatar visually indicates a third set of clinical values describing the physiological subsystem of the subject.

In this example, the second avatar is a graphical depiction of the subject's heart, and the third set of clinical values are a set of cardiac values of the subject. Further, in this example, the second set of clinical values is a subset of the third set of clinical values. Thus, when it is predicted that the condition of the subject's cardiac system is likely to change, a display is controlled to show a set of detailed cardiac parameters, including those used in the determination that the predetermined condition is met, to permit close monitoring of the system.

The modification to the first avatar comprises a modification to be made to a graphical property of the first avatar. The graphical property of the first avatar comprises at least one of: a dimension of the first avatar; a scale of the first avatar; a position of a first avatar; an orientation of the first avatar; a color of the first avatar; a color of a perimeter region of the first avatar; and a transparency of the first avatar. Thus, the modification to the first display control signal results in some modification to the first avatar which may result in drawing attention away from the first avatar and towards the second avatar.

The method 100 presents just one way of determining whether a predetermined condition is met which uses a predictive model, and this may be implemented differently in alternative embodiments.

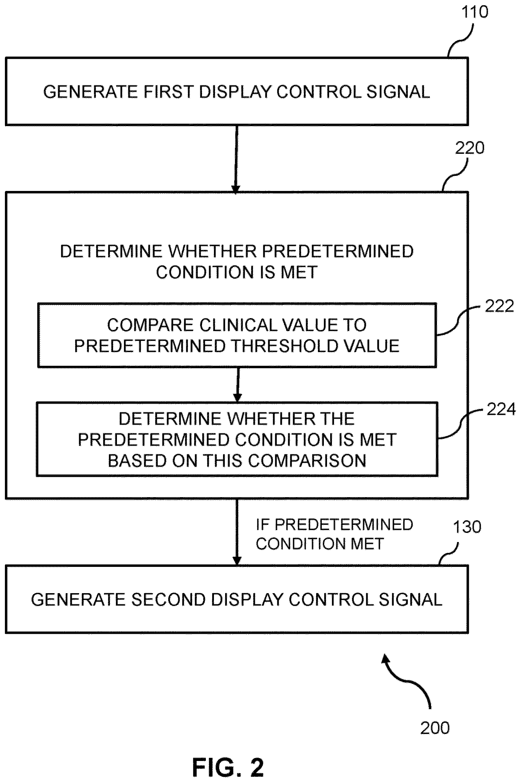

Referring now to Fig. 2 there is depicted a simplified diagram of a computer-implemented method 200 for controlling a display according to another proposed embodiment.

Steps 110 and 130 of the method 200 are identical to the steps 110 and 130 of the method 100 and therefore a detailed discussion of these steps will not be repeated. The method 200 differs from the method 100 in that step 120 is replaced by a step 220 comprising sub-steps 222 and 224.

Step 222 comprises comparing a clinical value of the second set of clinical values to a predetermined threshold value. Step 224 comprises determining whether a predetermined condition is met based on this comparison. Thus, the method 200 involves modifying a display control signal if a cardiac parameter value falls outside of a predetermined range of values that are known to indicate a healthy and/or stable subject state.

Other implementations of the predetermined condition are also possible. For example, determining whether a predetermined condition is met may comprise: analyzing the second set of clinical values to determine the rate of change of a value of a parameter of the physiological subsystem; and determining whether the predetermined condition is met based on the determined rate of change of the value of the parameter of the physiological subsystem.

In some embodiments, the determination of whether the predetermined condition is met may further incorporate determining whether certain sensors are active or attached to a subject monitoring display and therefore which clinical values are available for display. Certain method of predicting subject instability or deterioration in condition do not require advanced clinical parameters. For example, a hemodynamic stability index (HSI) may be used to predict from a combination of subject's vital signs, subject characteristics, and ventilator parameters, whether a subject is heading towards hemodynamic instability and therefore the HSI having a certain value may be an example of a predetermined condition in the proposed invention. However, the evaluation of the HSI does not require sensors for advanced hemodynamic parameters to be actively monitoring the subject. Therefore, in this instance switching to a second avatar that shows the hemodynamic system of the subject would provide no further information than the general first avatar of the subject. Therefore, for the predetermined condition to be met, it may be further required that certain sensors or measurement devices are active.

Similarly other aspects of the method 100 and 200 may be implemented differently in alternative embodiments. For example, although in the methods 100 and 200, modification of the first avatar comprises a modification of a graphical property of the first avatar, in other embodiments this need not be the case. For example, in some proposed embodiments modification to the first avatar comprises removing the first avatar entirely and replacing it with the second avatar.

Methods 100 and 200 are discussed in the context of a subject's cardiac system but the concepts of the proposed methods apply equally well to other physiological subsystems. Examples of possible physiological subsystems include: the pulmonary system; the cardiopulmonary system; the neurological system; the digestive system; the urinary system; the immune system; the endocrine system; the thermoregulatory system; and/or separate organs, such as the kidneys or the heart.

The first avatar and second avatar may include any visual means for depicting the first and third sets of clinical values and therefore do not necessarily comprise graphical depictions of the subject's body parts. For example, in some embodiments, an avatar may comprise a graph or chart displaying the values of the third set of clinical values and optionally showing how these values are changing in real-time. The avatars may contain icons, alarms, waveforms, or analytics to present the sets of clinical values.

The second set of clinical values need not comprise a subset of the third set of clinical values. The second set of clinical values may in some embodiments comprise a subset of the first set of clinical values. In other embodiments, the first, second, and third sets of clinical values have no overlap and relate to different physiological parameters of the subject. In other embodiments, the clinical values represented by the second avatar may comprise: a selection of the clinical values of the first set of clinical values that relate to the physiological subsystem; a selection of the second set of clinical values used to determine if the predetermined condition is met; and a selection of additional clinical values that provide a more detailed description of the physiological subsystem. The first, second, and third sets of clinical values may each comprise a combination of measured values and intrinsic properties of the subject (for example demographic parameters or pre-known medical conditions).

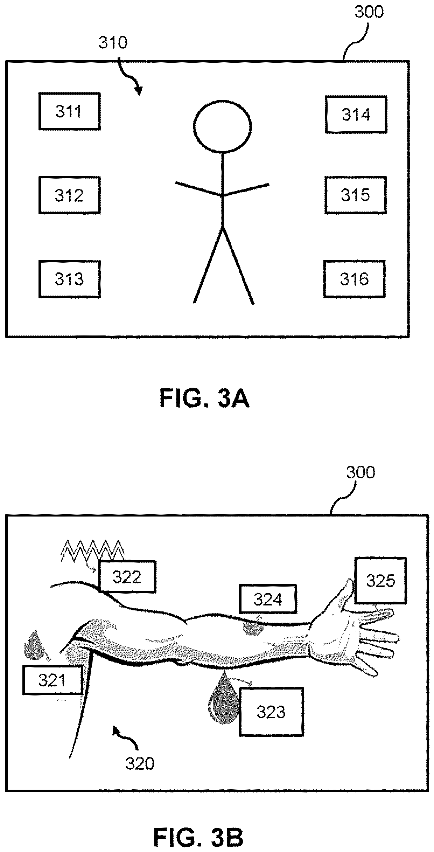

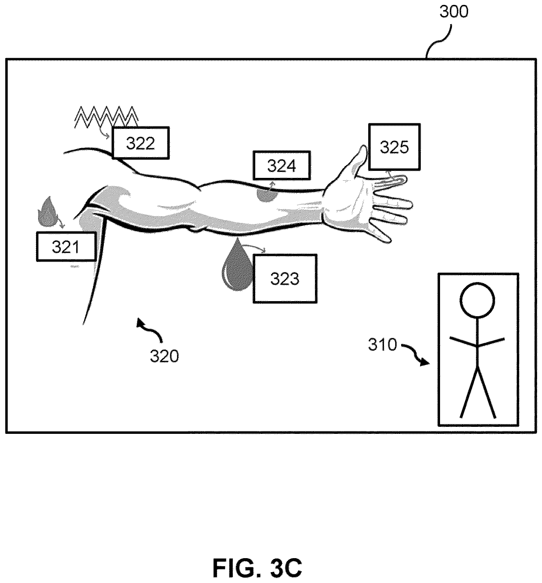

Figs. 3A, 3B, and 3C each depict a schematic illustration of a subject monitoring display 300 controlled according to a proposed method of the present invention.

Fig. 3A depicts the subject monitoring display 300 when it is controlled by a first display control signal. The subject monitoring display is controlled to depict a first avatar 310 comprising a graphical representation of the subject's whole body. The first avatar also visually indicates a first set of clinical values 311, 312, 313, 314, 315, and 316.

Fig. 3B depicts the subject monitoring display 300 when it is controlled by the second display control signal. The subject monitoring display is controlled to remove the depiction of the first avatar and replace it with a second avatar 320. The second avatar visually indicates a third set of clinical parameters (321, 322, 323, 324, 325, and 323), and a physiological subsystem of the subject. In this exemplary embodiment the physiological subsystem is the thermoregulatory system of the subject, and the second set of clinical values comprises values such as core temperature, skin temperature, shivering level, perfusion index, and sweat rate.

Fig. 3C depicts the subject monitoring display 300 when it is controlled by an alternative second display control signal to that associated with Fig. 3B. In this embodiment, the second display control signal describes a modification to a graphical property of the first avatar rather than the complete removal of the depiction of the first avatar. Thus, in this embodiment, the first avatar 310 is depicted at a reduced scale in the bottom right hand corner of the display whilst the second avatar 320 is depicted at a larger scale. The first avatar 310 may also be simplified slightly to allow it still to be legible in the reduced scale.

Figs. 3B and 3C show just two examples of how the second display control signal may control the display 300 such that when a certain predetermined condition is met the display prominently depicts salient clinical values for assessing the health condition of the subject. Many alternative implementations are possible. For example, the determination of whether the first condition is met may result in a determination that both the first avatar and the second avatar are of similar importance. In this case, the second display control signal may control the display to show both avatars alongside each other having a similar size.

In some proposed methods, determining whether the predetermined condition is met may comprises analyzing a plurality of clinical values describing a plurality of different physiological sub-systems of the subject. In this instance, determining whether the predetermined condition is met may comprise determining whether any of the plurality of different physiological sub-systems are in (or are likely to be in) an alarm state. The second display control signal may then comprise a plurality of second avatars which each visually depict a set of clinical values describing a physiological sub-system of the plurality of physiological sub-systems that has been determined to be in an alarm state. In this instance, each avatar described by the second display control signal may have a similar size or may have different sizes depending on how important the information in a particular avatar is deemed to be for assessing the health state of the subject. Alternatively, the relative importance of each avatar may be indicated by its position or the color of its border or perimeter area. This ranking of the importance of particular avatars may be based on how many of the clinical values associated with each avatar are in an alarm range, although more complicated algorithms could also be used.

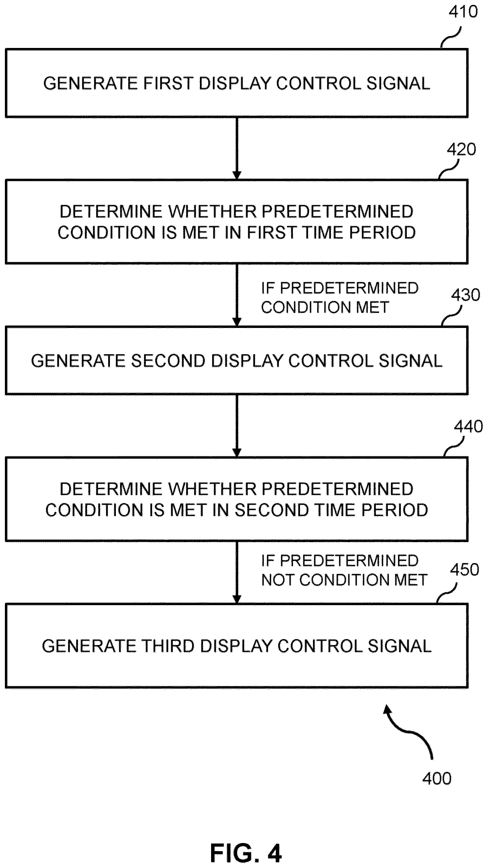

Referring now to Fig. 4, there is depicted a simplified flow diagram of a computer-implemented method 400 for controlling a display according to a third proposed embodiment.

The method 400 commences with a step 410 comprising generating a first display control signal for controlling a display, the first display control signal describing a first avatar of the subject, wherein the first avatar visually indicates a first set of clinical values of the subject.

The method then proceeds to step 420 comprising determining whether a predetermined condition is met based on a second set of clinical values of the subject, wherein the second set of clinical values describe a physiological subsystem of the subject. In this exemplary embodiment, the second set of clinical values describes at least one time varying parameter of the physiological system, and determining whether the predetermined condition is met comprises determining whether the predetermined condition is met during a first time period.

If the predetermined condition is met in the first time period, the method proceeds to step 430 comprising modifying the first display control signal to generate a second display control signal, the second display control signal describing a modification to the first avatar, and a second avatar of the subject, wherein the second avatar visually indicates a third set of clinical values describing the physiological subsystem of the subject.

The method 400 further comprises step 440 comprising determining, based on the second set of clinical values, whether the predetermined condition is met in a second time period that commences later than the first time period.

If the predetermined condition is not met in the second time period, the method proceeds to step 450 comprising modifying the second control display signal to generate a third control display signal describing a modification to the second avatar, and the first avatar of the subject.

Thus, the method 400 may involve modifying the view of a controlled display once the predetermined condition is no longer met. This may involve switching back to a depiction of only the first avatar to allow for more general monitoring of the subject. The second time period may be configured to be offset in time from the first time period by at least a minimum time interval, such that the display only switches back to the first avatar after this time period has passed and the subject is stable. This may help to prevent rapid switching between different views.

Alternatively, or in addition, in some proposed embodiments the second display control signal may also be modified if one or more of the first set of clinical values that is not present in the third set of clinical values enters an alarm range and/or changes rapidly in the second time period even if the predetermined condition is still met. Thus, in some embodiments, the method may further comprise determining, based on the first set of clinical values, whether an alternative predetermined condition is met in the second time period; and if the alternative predetermined condition is met, modifying the first display control signal to generate a fourth display control signal describing a modification to the first avatar. This may involve the first avatar reappearing on the display, or the depiction of the first avatar (if it is already present on the display) being enlarged or modified in some way (for example with a flashing effect), so as to highlight that one of the first clinical values is in an alarm range and/or is changing rapidly.

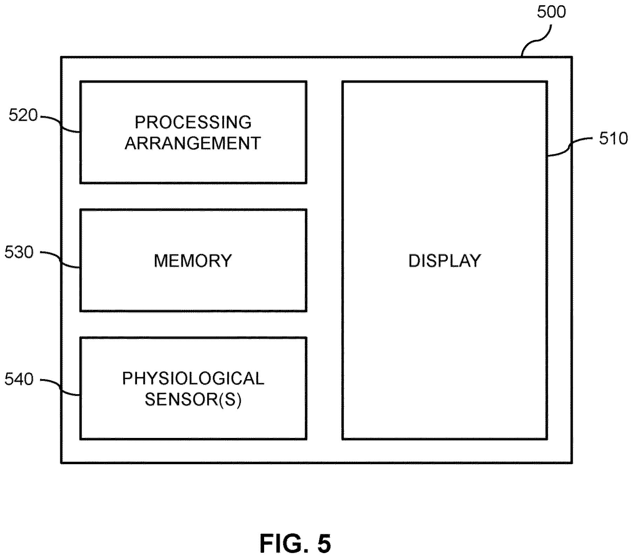

Fig. 5 depicts a simplified block diagram of a system 500 for displaying clinical values of a subject according to an aspect of the present invention. The system 500 comprises a display 510, a processing arrangement 520, a memory 530 and one or more physiological sensors 540.

The one or more physiological sensors 540 are configured to obtain measurements of at least one clinical value. As an example, this may include pulse oximeter measurements, blood pressure measurements, heart rate measurements, ECG measurements, and breathing rate measurements. However, the present invention is not limited to such and the one or more physiological sensor 540 may comprise any suitable known or novel sensors for measuring a clinical parameter of the subject. Further clinical values of the subject, including historic measurements of clinical values and/or intrinsic clinical parameters of the subject (e.g., subject age and gender, and whether they have any pre-existing medical conditions) may be stored as part of the subject's medical record in the memory 530.

The processing arrangement 520 is configured to generate a first display control signal for controlling the display 510. The first display control signal describes a first avatar of the subject, wherein the first avatar visually indicates a first set of clinical values of the subject. The processing arrangement 520 is further configured to determine whether a predetermined condition is met based on a second set of clinical values of the subject, wherein the second set of clinical values describe a physiological subsystem of the subject. If the predetermined condition is met, the processing arrangement 520 modifies the first display control signal to generate a second display control signal, the second display control signal describing a modification to the first avatar, and a second avatar of the subject, wherein the second avatar visually indicates a third set of clinical values describing the physiological subsystem of the subject. It will be understood that the first second, and third subsets of clinical values may each comprise at least one of: a value acquired via the one or more physiological sensors 540; and a value stored in the memory 530.

The first and second display control signals generated by the processing arrangement 520 in this manner are then used to control the output of the display 510 to control which clinical values are presented on the display 510. The selection of certain clinical values in this way may aid a health professional to accurately monitor and assess the health status of a subject.

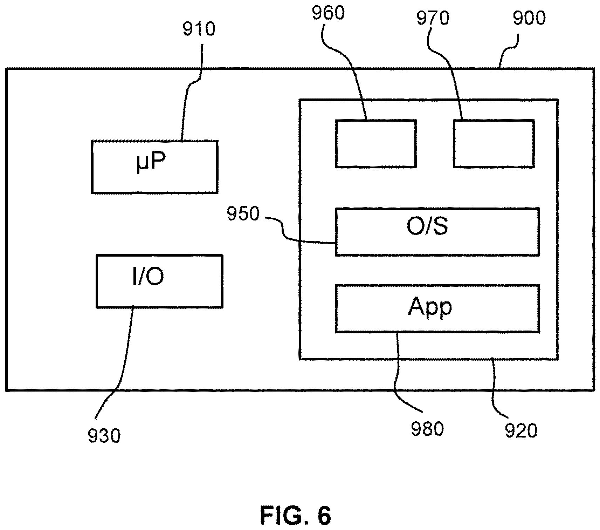

Fig. 6 illustrates an example of a computer 900 within which one or more parts of an embodiment may be employed. Various operations discussed above may utilize the capabilities of the computer 900. In this regard, it is to be understood that system functional blocks can run on a single computer or may be distributed over several computers and locations (e.g., connected via the internet).

The computer 900 includes, but is not limited to, PCs, workstations, laptops, PDAs, palm devices, servers, storages, and the like. Generally, in terms of hardware architecture, the computer 900 may include one or more processors 910, memory 920 and one or more I/O devices 930 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 910 is a hardware device for executing software that can be stored in the memory 920. The processor 910 can be virtually any custom made or commercially available processor, a central processing unit (CPU), a digital signal processor (DSP), or an auxiliary processor among several processors associated with the computer 900, and the processor 910 may be a semiconductor based microprocessor (in the form of a microchip) or a microprocessor.

The memory 920 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 920 may incorporate electronic, magnetic, optical, and/or other types of storage media. Note that the memory 920 can have a distributed architecture, where various components are situated remote from one another, but can be accessed by the processor 910.

The software in the memory 920 may include one or more separate programs, each of which comprises an ordered listing of executable instructions for implementing logical functions. The software in the memory 920 includes a suitable operating system (O/S) 950, compiler 960, source code 970, and one or more applications 980 in accordance with exemplary embodiments. As illustrated, the application 980 comprises numerous functional components for implementing the features and operations of the exemplary embodiments. The application 980 of the computer 900 may represent various applications, computational units, logic, functional units, processes, operations, virtual entities, and/or modules in accordance with exemplary embodiments, but the application 980 is not meant to be a limitation.

The operating system 950 controls the execution of other computer programs, and provides scheduling, input-output control, file and data management, memory management, and communication control and related services. It is contemplated by the inventors that the application 980 for implementing exemplary embodiments may be applicable on all commercially available operating systems.

Application 980 may be a source program, executable program (object code), script, or any other entity comprising a set of instructions to be performed. When a source program, then the program is usually translated via a compiler (such as the compiler 960), assembler, interpreter, or the like, which may or may not be included within the memory 920, so as to operate properly in connection with the O/S 950. Furthermore, the application 980 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 930 may include input devices such as, for example but not limited to, a mouse, keyboard, scanner, microphone, camera, etc. Furthermore, the I/O devices 930 may also include output devices, for example but not limited to a printer, display, etc. Finally, the I/O devices 930 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 930 also include components for communicating over various networks, such as the Internet or intranet.

If the computer 900 is a PC, workstation, intelligent device or the like, the software in the memory 920 may further include a basic input output system (BIOS) (omitted for simplicity). The BIOS is a set of essential software routines that initialize and test hardware at start-up, start the O/S 950, and support the transfer of data among the hardware devices. The BIOS is stored in some type of read-only-memory, such as ROM, PROM, EPROM, EEPROM or the like, so that the BIOS can be executed when the computer 900 is activated.

When the computer 900 is in operation, the processor 910 is configured to execute software stored within the memory 920, to communicate data to and from the memory 920, and to generally control operations of the computer 900 pursuant to the software. The application 980 and the O/S 950 are read, in whole or in part, by the processor 910, perhaps buffered within the processor 910, and then executed.

When the application 980 is implemented in software it should be noted that the application 980 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 980 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods of Figs. 1, 2, and 4 and the system of Fig. 5 may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Figs. 5 and 6 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems.

The flowcharts and block diagrams in the Figures illustrate the architecture, functionality, and operation of possible implementations of systems, methods, and computer program products according to various embodiments of the present invention. In this regard, each block in the flowcharts or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustrations, and combinations of blocks in the block diagrams and/or flowchart illustrations, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention.

Variations to the disclosed embodiments can be understood and effected by those skilled in the art in practicing the claimed invention from a study of the drawings the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If the term "adapted to" is used in the claims or description it is noted the term "adapted to" is intended to be equivalent to the term "configured to". Any reference signs in the claims should not be construed as limiting the scope.

## Claims

1. A computer-implemented method for controlling a display, the method comprising:
generating a first display control signal for controlling a display, the first display control signal describing a first avatar of the subject, wherein the first avatar visually indicates a first set of clinical values of the subject;
determining whether a predetermined condition is met based on a second set of clinical values of the subject, wherein the second set of clinical values describe a physiological subsystem of the subject; and
if the predetermined condition is met, modifying the first display control signal to generate a second display control signal, the second display control signal describing a modification to the first avatar, and a second avatar of the subject, wherein the second avatar visually indicates a third set of clinical values describing the physiological subsystem of the subject.

2. The method of claim 1, wherein an avatar comprises a graphical representation of at least one body part of the subject.

3. The method of claim 2, wherein the first avatar comprises a graphical representation of the subject's whole body and the second avatar comprises a graphical representation of the physiological subsystem of the subject.

4. The method of any preceding claim, wherein a modification of the first avatar comprises at least one of:
a modification to be made to a graphical property of the first avatar; and
removal of the first avatar.

5. The method of claim 4, wherein a graphical property of the first avatar comprises at least one of:
a dimension of the first avatar; a scale of the first avatar;
a position of a first avatar;
an orientation of the first avatar;
a color of the first avatar;
a color of a perimeter region of the first avatar; and
a transparency of the first avatar.

6. The method of any preceding claim, wherein determining whether a predetermined condition is met comprises:
processing the second set of clinical values with a predictive model to predict a future condition of the physiological subsystem; and
determining whether the predetermined condition is met based on the predicted future condition.

7. The method of any preceding claim, determining whether a predetermined condition is met comprises:
comparing a clinical value of the second set of clinical values to a predetermined threshold value; and
determining whether the predetermined condition is met based on this comparison.

8. The method of any preceding claim, wherein determining whether a predetermined condition is met comprises:
analyzing the second set of clinical values to determine the rate of change of a value of a parameter of the physiological subsystem; and
determining whether the predetermined condition is met based on the determined rate of change of the value of the parameter of the physiological subsystem.

9. The method of any preceding claim wherein the first set of clinical values comprises a plurality of vital sign measurements of the subject and optionally, wherein the plurality of vital sign measurements comprise at least one of:
a blood oxygen saturation value (SpO2);
a heart rate value;
a respiration rate value;
an ST elevation value;
a blood pressure value;
a central venous pressure value;
an end-tidal carbon dioxide value;
a body temperature value;
a cardiac output value;
a brain activity value;
a relaxation status value;
a fraction of inspired oxygen (FiO2) value;
an airway pressure value; and
a tidal volume.

10. The method of any preceding claim wherein the second set of clinical values comprises a subset of the third set of clinical values.

11. The method of any preceding claim, wherein the second set of clinical values describes at least one time varying parameter of the physiological system and determining whether the predetermined condition is met comprises determining whether the predetermined condition is met during a first time period.

12. The method of claim 11, wherein if the predetermined condition is met in the first time period the method further comprises:
determining, based on the second set of clinical values, whether the predetermined condition is met in a second time period that commences later than the first time period; and
if the predetermined condition is not met in the second time period, generating a third control display signal describing a modification to the second avatar, and the first avatar of the subject.

13. A computer program comprising computer program code means adapted, when said computer program is run on a computer, to implement the method of any of claims 1-12.

14. A processing arrangement configured to:
generate a display control signal for controlling a display, the display control signal describing a first avatar of the subject, wherein the first avatar visually indicates a first set of clinical values of the subject;
determine whether a predetermined condition is met based on a second set of clinical values of the subject, wherein the second set of clinical values describe a physiological subsystem of the subject; and
if the predetermined condition is met, modify the first display control signal to generate a second display control signal, the second display control signal describing a modification to the first avatar, and a second avatar of the subject, wherein the second avatar visually indicates a third set of clinical values describing the physiological subsystem of the subject.

15. A system for displaying clinical values, the system comprising:
a display; and
the processing arrangement 13 for controlling the display.
